# EUROPEAN PATENT SPECIFICATION

(11) **EP 0 998 228 B1**
(45) Date of publication and mention of the grant of the patent: **16.06.2004**
(21) Application number: 98935918.7
(22) Date of filing: 23.07.1998
(51) Int. Cl.: A61B 17/22

(54) **ROTATIONAL THROMBECTOMY APPARATUS WITH STANDING WAVE**
ROTIERENDE THROMBEKTOMIEVORRICHTUNG MIT STEHENDER WELLE
APPAREIL ROTATIF DE THROMBECTOMIE A ONDE STATIONNAIRE

(30) Priority: 24.07.1997 US 53475 P
(43) Date of publication of application: 10.05.2000
(73) Proprietor: Rex Medical, L.P., Radnor, PA 19087 (US)
(72) Inventor: McGuckin, James, F., Jr., Radnor PA 19087 (US)
(74) Representative: Jackson, Derek Charles
(86) International application number: PCT/US1998/015156
(87) International publication number: WO 1999/004701

(56) References cited:
- EP-A- 0 177 782
- EP-A- 0 452 631
- EP-A- 0 709 110
- DE-U- 8 900 494
- US-A- 4 883 460
- US-A- 5 248 296

## Description

### Field of the Invention

This invention relates to rotational thrombectomy apparatus in the form of apparatus for clearing a body lumen of thrombolytic material. Such apparatus may be used for clearing recurring thrombosis of hemodialysis grafts.

### Background of the Invention and Description of the Prior Art

Modern hemodialysis technology enables patients with chronic renal failure to live independently between dialysis treatments. Patients utilize this technology as a means of filtering the toxins from their blood by passing blood out of their body through a hemodialysis machine. The hemodialysis machine removes blood toxins by exposing the blood to dialyzing fluid across a semipermeable membrane, in effect creating an artificial kidney.

In order to properly process a patient's blood a graft is made, preferably in the patient's arm. At the site of the graft a shunt is placed to connect an artery having a high rate of blood flow with a vein. The shunt provides a convenient inlet on the artery side for blood requiring dialysis filtration processing; the outlet is located on the vein side for return of dialysis processed blood from the hemodialysis station.

The dialysis shunt, while providing a convenient arrangement for hemodialysis processing, may become inoperable after a period of time due to stenosis caused by the high rate of blood flow through the shunt and repetitive injury at the venous anastomosis. Typically, patients must have these constricting portions of the shunt widened periodically in order to continue hemodialysis processing through the shunt.

Shunt blockage is generally treated through a combination of surgical devices and/or pharmaceutical treatments; these techniques are often cost prohibitive and/or require an incision. For example, pharmaceutical treatments generally employ urokinase which, depending on the amount used, can cost upward of $350.00 per application and possible cause bleeding complications.

Mechanical thrombolysis apparatus and methods for performing thrombolysis are known, being disclosed in United States patents 4,646,736 to Auth, 5,078,722 to Stevens, 5,695,507 to Auth, et al and EP-A-0 177 782.

The apparatus disclosed in these patents seeks to penetrate thrombolytic structures by introducing, for example in the case of '507 and EP-A-0 177 782, a rotating core wire into the thrombus, seeking to withdraw fibrin from the thrombus into the rotating core wire thereby breaking up the network of the thrombus which is preventing blood flow.

### Summary of the Invention

According to the present invention there is provided apparatus for clearing a body lumen of thrombolytic material comprising one hand manipulable means for providing rotary motion output, an elongated wire connected to the rotary motion output means for rotation thereby, a catheter for enveloping a length of the wire, and gripping means facilitating manual rotation of the catheter by the one hand independently of the wire, wherein the elongated wire is flexible and the rotary motion output means is adapted to rotate the wire at a speed sufficient to cause the wire to flex and to create a standing wave in a portion of the wire extending from the catheter to liquefy thrombolytic material.

The means for providing rotary motion output may comprise a motor including control means. A housing may be provided for the motor and for the control means, adapted for grasping by the one hand, the wire passing through the housing and emerging from opposite sides thereof.

The extremity of the wire remote from the catheter may be angularly disposed with respect to the remainder of the wire. Alternatively, the extremity of the wire remote from the catheter may be J-shaped. As a further alternative, the extremity of the catheter remote from the means for providing rotary motion output may be angularly disposed with respect to the remainder of the catheter.

The catheter may be sufficiently resistant to twisting that torque applied manually to the catheter proximate the means for providing rotary motion output results in corresponding angular rotary motion of the extremity of the catheter remote from the means.

Means may be provided communicating with the interior of the catheter for selectably supplying or exhausting fluid to and from the catheter interior. The gripping means may be fixedly connected to the means communicating with the interior of the catheter. The gripping means may be rotatable unitarily with the catheter about the wire.

A sheath may be provided enveloping the catheter and a conduit communicating with the interior of the sheath for selectably supplying or exhausting fluid to and from the sheath interior, externally of the catheter. The conduit may be movable longitudinally along the wire with the sheath. The means providing rotary motion output, the catheter, the wire, the means communicating with the interior of the catheter, the sheath and the conduit communicating with the interior of the sheath may all be manually disassemblable from one another, preferably manually disassemblable without the use of tools.

Manually actuable means may be provided for selectably connecting the wire to the means for providing rotary motion output.

The wire may be hydrophilic.

The wire may be braided. In such case, the wire may be rotatable in a direction to resist untwisting of the braid. Alternatively, the wire may be a single filament.

The wire may not be permanently deformable at room temperature.

The wire may be rotatable at a speed from 100 revolutions per minute to 10,000 revolutions per minute.

The apparatus may be used for clearing a surgical shunt connecting an artery to a vein and associated portions of the artery and vein of thrombolytic material by initially inserting a needle through a patient's skin and into the shunt, inserting a first wire through the needle, sensing whether the first wire is in the shunt preferably by tactile sensation received via the wire, x-ray inspecting skin preferably where the needle was inserted to determine whether the wire is within the shunt, removing the needle when the wire is determined to be within the shunt, placing a first catheter over the first wire with a catheter discharge orifice within the shunt, removing the first wire leaving the first catheter with the discharge end within the shunt, inserting a second wire through the first catheter into the interior of the shunt, removing the first catheter from the shunt, inserting a sheath over the second wire and into the shunt, removing the second wire, inserting a rotatable instrument wire and an associated surrounding catheter through the sheath and into the shunt, supplying lubricating fluid to the associated catheter interior to lubricate the instrument wire for rotation within the associated catheter, sweepingly rotating the instrument wire, but not the associated catheter, through the shunt to liquify thrombolytic material therein, removing the instrument wire and associated catheter from the sheath, applying suction to the sheath to remove liquid thrombolytic material from the shunt interior, injecting anti-coagulant into the shunt interior through the sheath, removing the instrument wire from the associated catheter, disconnecting the instrument wire from a drive motor associated therewith, re-inserting the instrument wire without the associated catheter through the sheath into the shunt interior and through any blockage at the vein end of the shunt and into the vein, placing an angioplasty balloon over the instrument wire, pushing the angioplasty balloon into position within venous anastomosis at vein-shunt juncture, removing the wire leaving the angioplasty balloon in position within the venous anastomosis at vein-shunt juncture, injecting radiology contrast dye through a lumen of the balloon vacated by removal of the instrument wire therefrom, observing dye travel through the vein to the patient's heart to reveal any venous blockages, inserting the instrument wire back into the balloon lumen, inflating the balloon to crush any venous anastomosis and open shunt-vein juncture, removing the balloon and instrument wire from the sheath, inserting a second sheath between the position of first sheath insertion and shunt-vein juncture into a shunt interior region cleansed of thrombolytic material, re-inserting the instrument wire without the associated catheter through the second sheath into the shunt through any blockage at shunt-artery juncture, placing an angioplasty balloon over the wire, pushing the balloon into position within arterial anastomosis at artery-shunt juncture, removing the wire leaving the angioplasty balloon in position, injecting contrasting radiology dye through the balloon lumen vacated by the wire, observing travel of the dye through the artery to the heart thereby revealing any arterial blockages, inserting the instrument wire back into the balloon lumen, inflating the balloon to crush any arterial anastomosis and thereby open shunt-artery juncture, removing a platelet plug and residual arterial anastomosis from the shunt-artery juncture by pulling on the balloon and, finally, removing the balloon, wire and sheath from the patient.

The apparatus may include a hydrophilic wire which is placed into the shunt access port, advanced to the blockage within a lubricated catheter which is steerable and is rotated, with the same hydrophilic wire extending beyond the tip of the directional catheter about its longitudinal axis thereby separating material adhering to the inside surface of the dialysis shunt and mechanically macerating the thrombus.

During rotation, the exposed length of the hydrophilic wire begins to periodically flex in an oscillatory fashion, forming a standing wave contributing to the ability to clear the length of the shunt. The maximum deflection points of the rotating wire allow shunt cleansing by rotating the oscillatory wire providing a scouring action clearing the wall of adherent thrombus while also breaking up clots within the graft. The tip of the hydrophilic wire is preferably translated into and out of the thrombus within the shunt thereby mechanically disrupting and dissolving clots in both directions of translation and hence decreasing time necessary to complete thrombolysis. The wire can then be disassembled from the device and used as necessary to complete a shunt renewal procedure.

The apparatus may also be used for clearing dialysis shunts. A catheter entrance port is prepared for reception of the hydrophilic wire apparatus. The tip of the wire is advanced into the clot; the wire is rotated preferably by an electric motor such that the hydrophilic wire rotates about its longitudinal axis within a lubricated catheter, with rotation of the wire creating an oscillatory flexing of the hydrophilic wire along substantially its length outside of the lubricating directional catheter.

### Brief Description of the Drawings

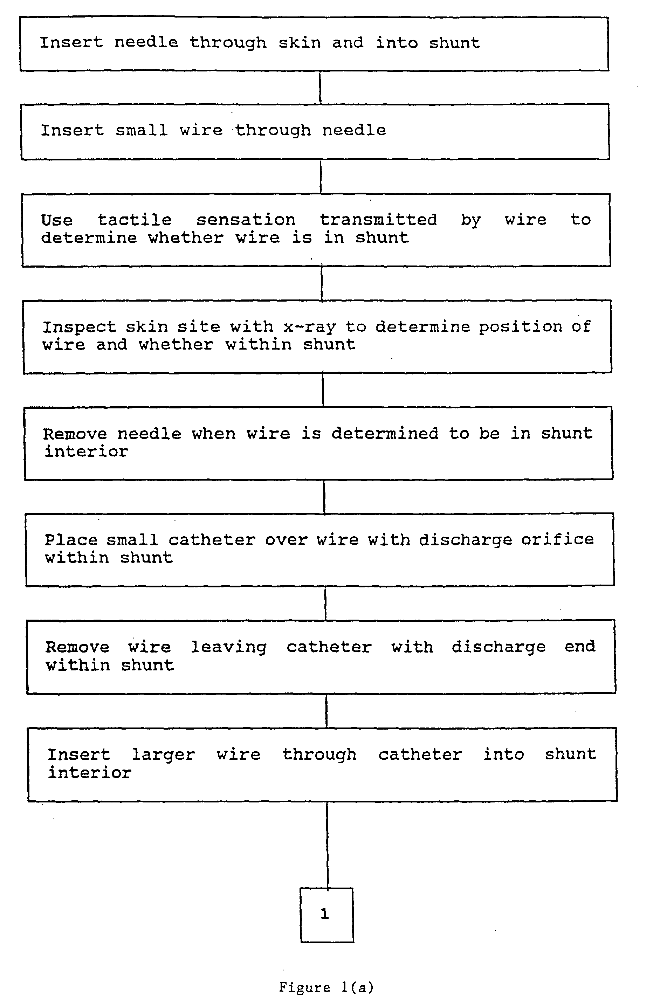

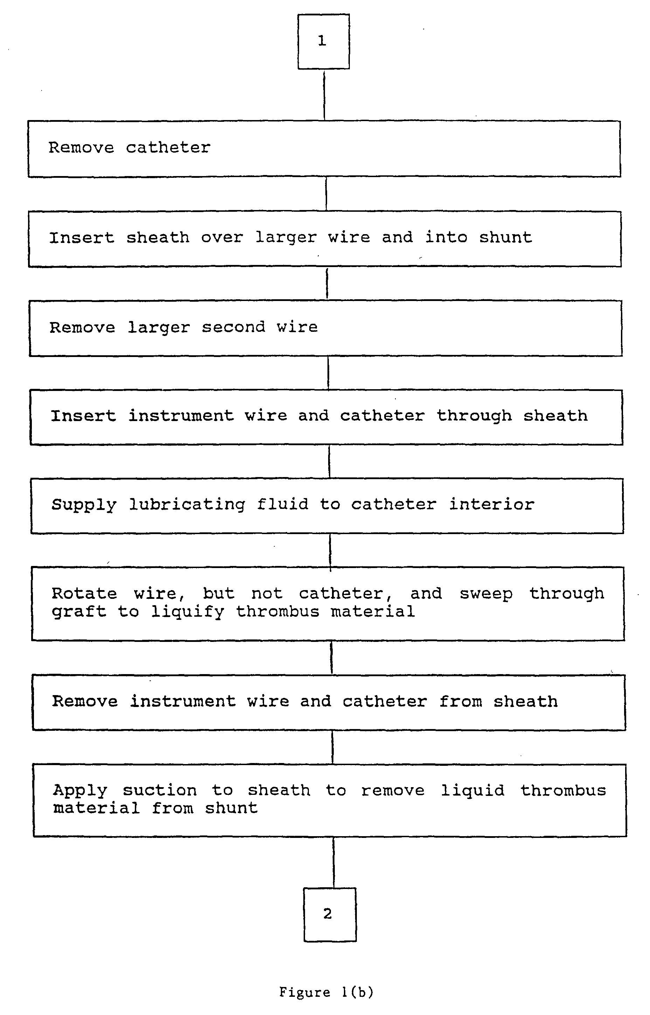

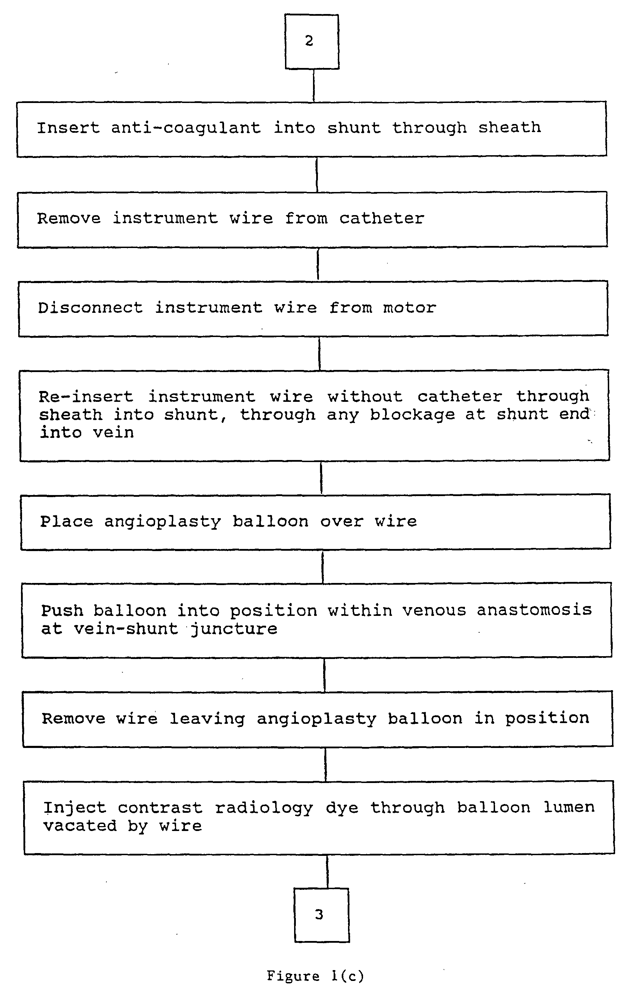

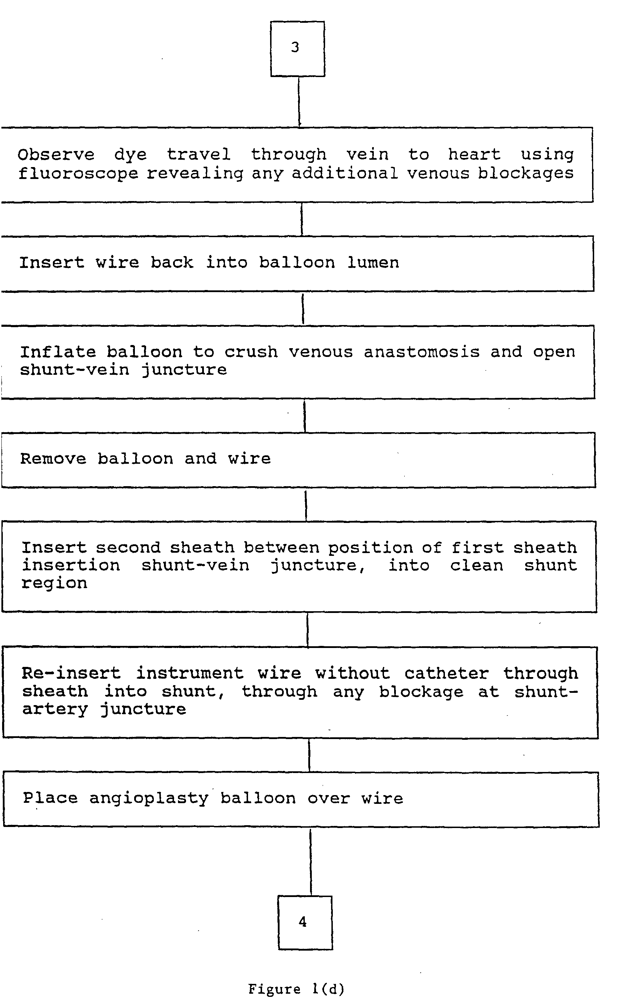

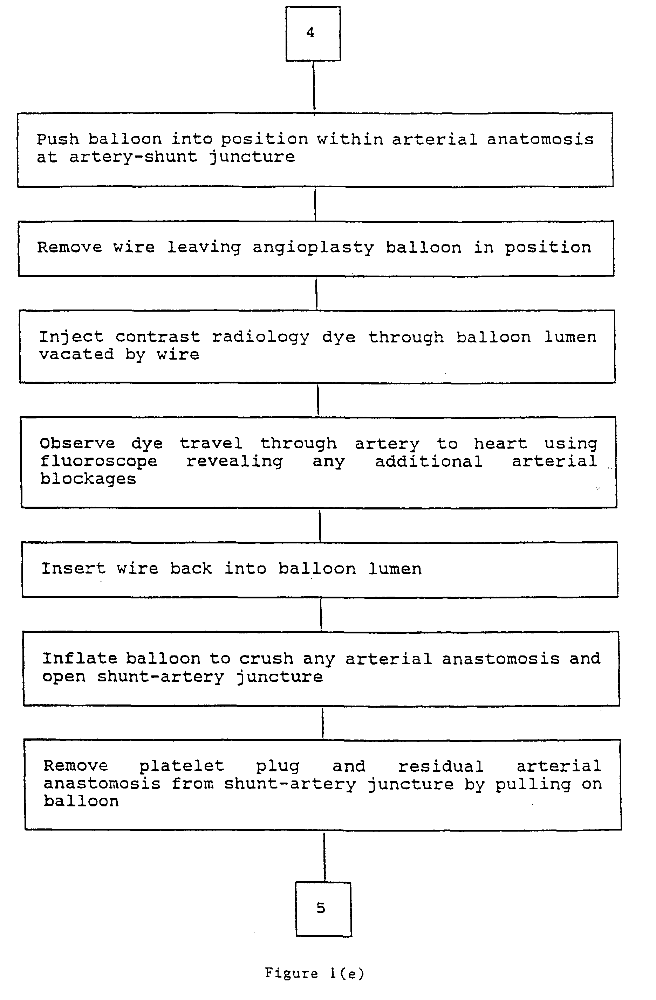

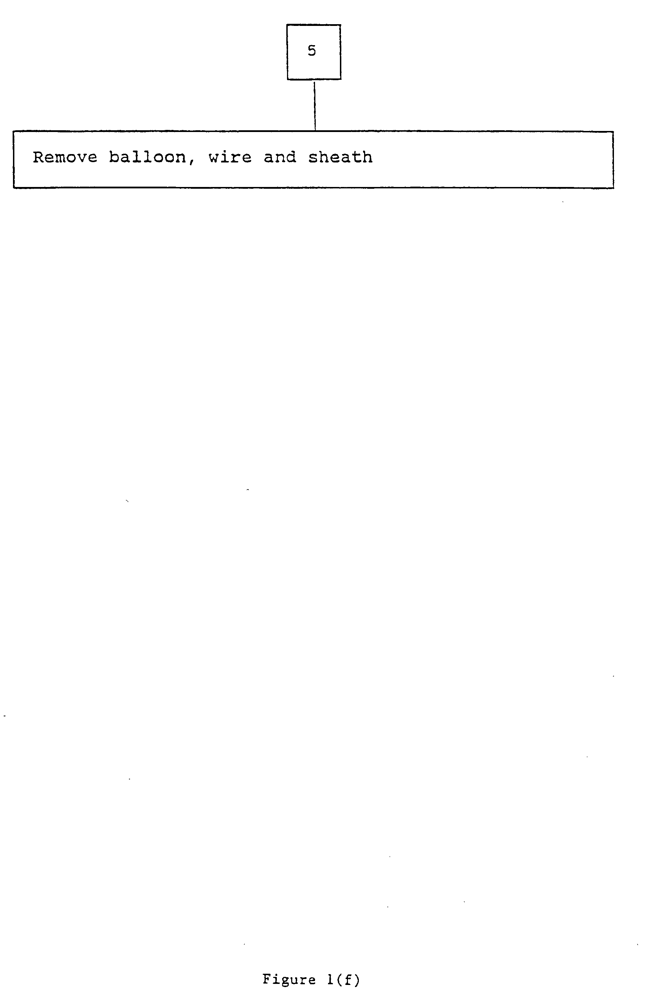

Figure 1 which is presented as Figures 1(a) through l(f) in separate pages, is a flow chart of a surgical method for using the apparatus of the invention;
Figure 2 is a partially exploded side sectional view of one embodiment of apparatus manifesting aspects of the invention;
Figure 3 is an unexploded top view of the apparatus shown in Figure 2;
Figure 4 is a top view of a second embodiment of apparatus in which the rotating wire extends through the handpiece;
Figure 5 illustrates apparatus in accordance with the invention in place within a dialysis shunt;
Figure 6 is a broken view illustrating an embodiment the catheter with a bent end to facilitate directional control of the wire;
Figure 7 is a schematic illustrating depicting hand operation of the apparatus in accordance with the invention;
Figure 8 depicts rotation of the wire in the catheter;
Figure 9 shows the standing wave formed by the wire resident in this shunt; and

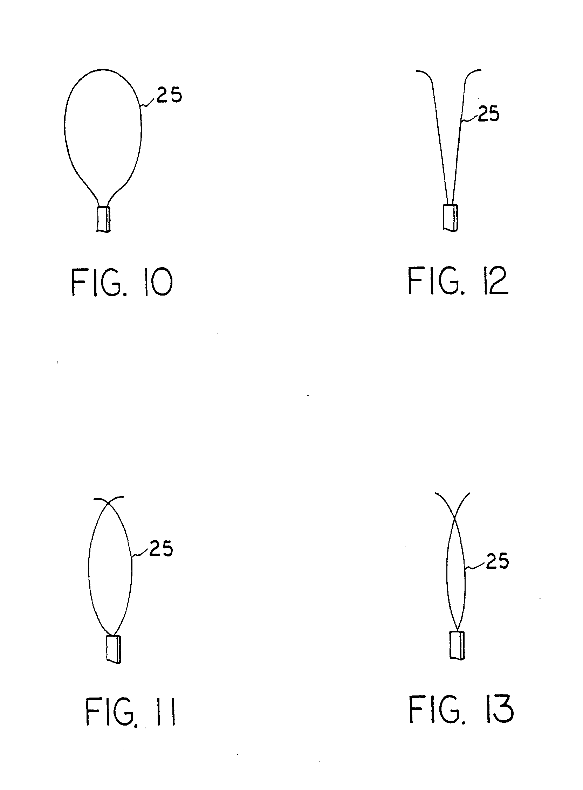

Figures 10 through 13 are alternative tip configurations of the rotating wire.

### Detailed Description of the Preferred Embodiment

This invention provides a surgical apparatus for clearing of dialysis shunt blockages in hemodialysis patients. Part of the surgical apparatus rotates, separating the blockage material from the inside surface of the dialysis shunt while macerating any thrombus within the shunt. Thus, the shunt is cleared with a minimum of trauma and without use of costly pharmaceuticals.

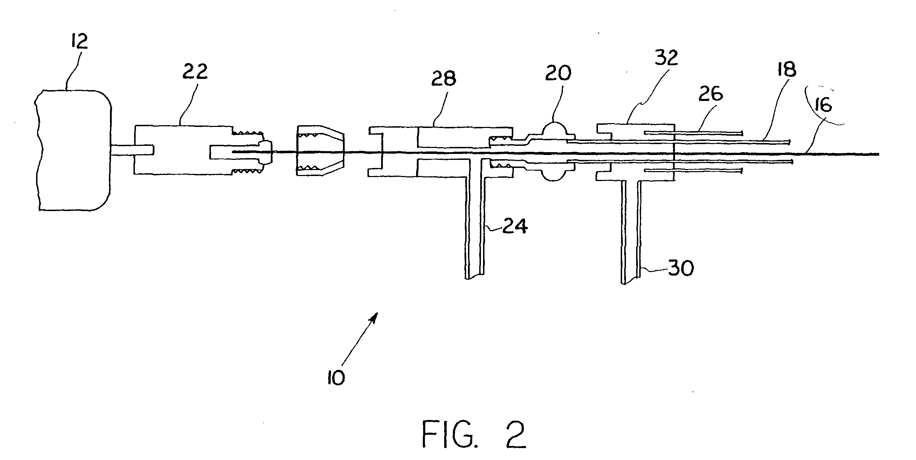

Referring to Figure 2 through 4, the surgical apparatus in accordance with the invention is generally designated 10 and includes a rotatable hydrophilic wire 16 and with a deformed tip 25.

Wire 16 rotates about an axis 11. Rotation of wire 16 of apparatus 10 is preferably performed by an electric motor 12, equipped with a mechanical hand control. However, wire 16 may be turned by pneumatic or hydraulic motor or even manually.

Hydrophilic wire 16 is preferably selected such that it rotates and oscillates so that a maximum number of points of maximum deflection between nodes of the standing wave reach the inner shunt wall to scour and remove adherent thrombus.

Apparatus 10 may be utilized to perform a number of procedures. Wire 16 is advanced through a catheter entrance port 52 of a dialysis shunt 34. Wire 16 is advanced along the interior surface of shunt 34 in the direction of a blockage; the tip of wire 16 may be translated into a thrombus, extending out of a distal tip of a directional lubricated catheter by the operator handling apparatus 10. As wire tip 25 rotates about axis 11, an adherent clot is separated from the interior surface 54 of dialysis shunt 34 by rotating contact of deformable wire tip 25 as well as oscillatory flexing of wire 16 in both directions along the longitudinal axis of the shunt as a standing wave (or vibrational node) is formed in wire 16.

The clot material is broken up by rotation of wire 16 sufficiently such that passage of clot material does not present a physiological problem for the patient; alternatively the clot material may be aspirated out of the shunt via an access port.

Referring to Figures 2, 3 and 4, motor 12 preferably includes control means for the motor which is operable using the hand which holds the motor. One hand preferably grasps motor 12 and operates the control means therefor. Elongated wire 16, which is also sometimes called the instrument wire, is connected to motor 12 for rotation thereof by motor 12. A catheter 18 envelops wire 16. The tubular gripping means 20 fits about catheter 18 to facilitate manual rotation of catheter 18. A manually operable chuck 22 provides means for selectively connecting wire 16 to motor 12.

A first conduit 24 is provided communicating with the interior of catheter 18 via a first fitting 28 which connects the first conduit to the interior of catheter 18. A second conduit 30 provides communication with the interior of sheath 26 via a second fitting 32 providing such connection. A surgical shunt 34 is provided between the vein and artery of the patient to undergo dialysis.

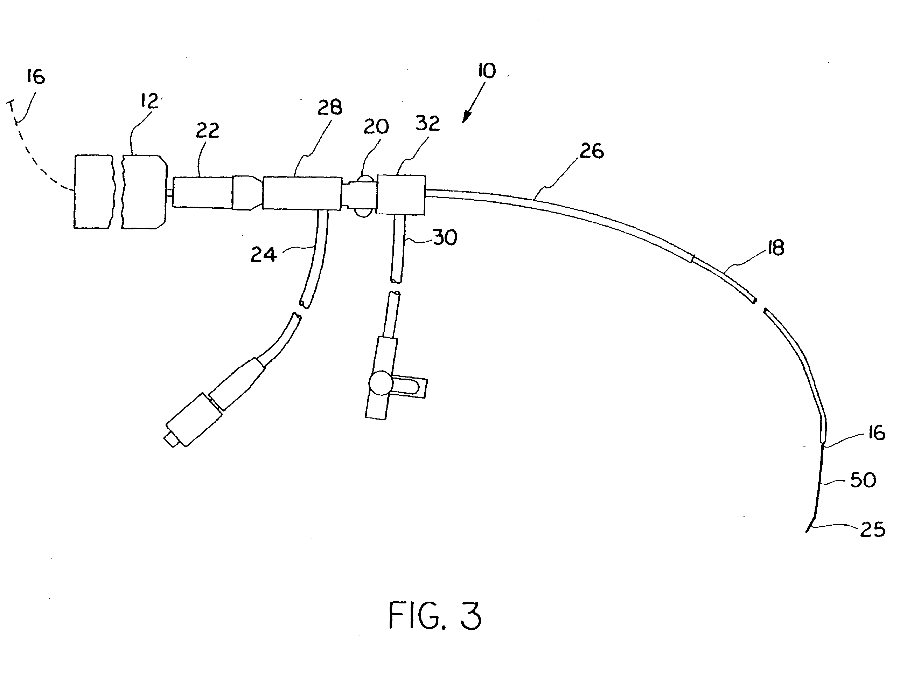

In use of the apparatus illustrated in Figures 2 and 3, motor 12 turns wire 16 while catheter 18 is rotated by manually actuating gripping means 20. Gripping means 20 together with fitting 28 is moveable axially along wire 16 to control the amount of wire 16 which is exposed beyond the extremity of catheter 18.

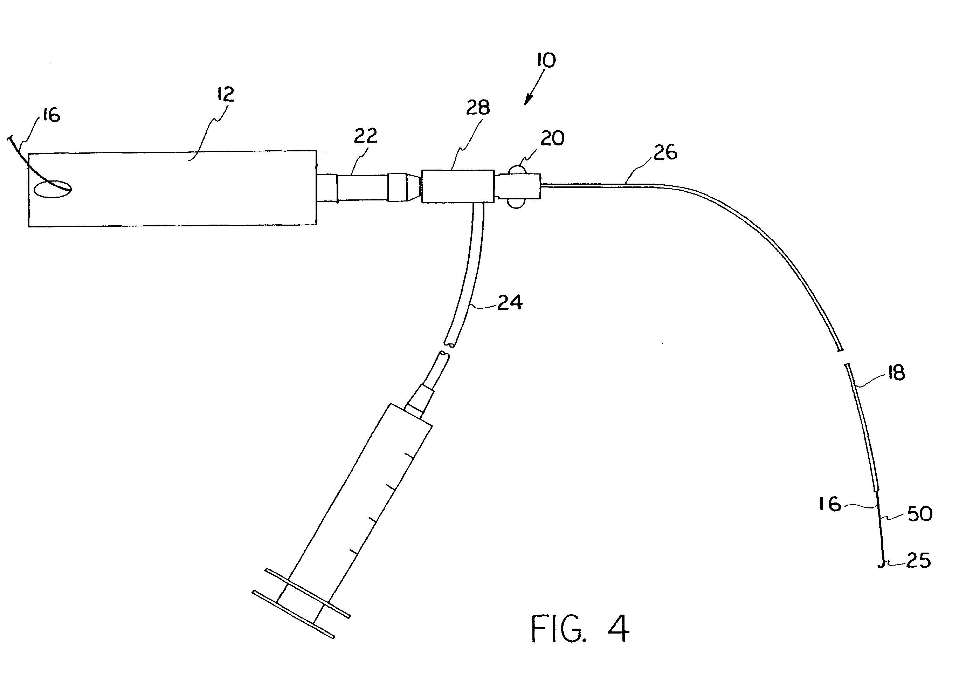

In the embodiment of the apparatus illustrated in Figure 4, wire 16 desirably extends out the rear of a housing for motor 12. This facilitates withdrawal of wire 16 to and from the shunt, artery and vein of interest.

In the embodiment illustrated in Figure 4, a second fitting 32 has not been provided nor has a second conduit been provided for input of fluid to the interior of sheath 26.

The portion of wire 16 which is exposed beyond the tip of catheter 18 is designated 50 in the drawings.

Not only is first fitting 28 and first conduit 24 moveable together with gripping means 20 and with catheter 18 relative to wire 16 but also second fitting 32 and second conduit 30 are preferably moveable with sheath 26 relative to wire 16.

The extremity of wire 16 remote from the catheter, denoted 50 in the drawings, may be angularly disposed with respect to the remainder of the wire. Alternatively, extremity 50 and wire 16 remote from the tip bend of the catheter may be J shaped.

The extremity portion of catheter 18 remote from motor 12 may be angularly disposed with respect to the remainder of the catheters; this configuration helps positioning of exposed portion of wire 16 by manual movement of catheter 18.

Preferably, second conduit 30, second fitting 32, sheath 26, motor 12, catheter 18, first conduit 24, fitting 28 and wire 16 are all manually disassemblable from one another.

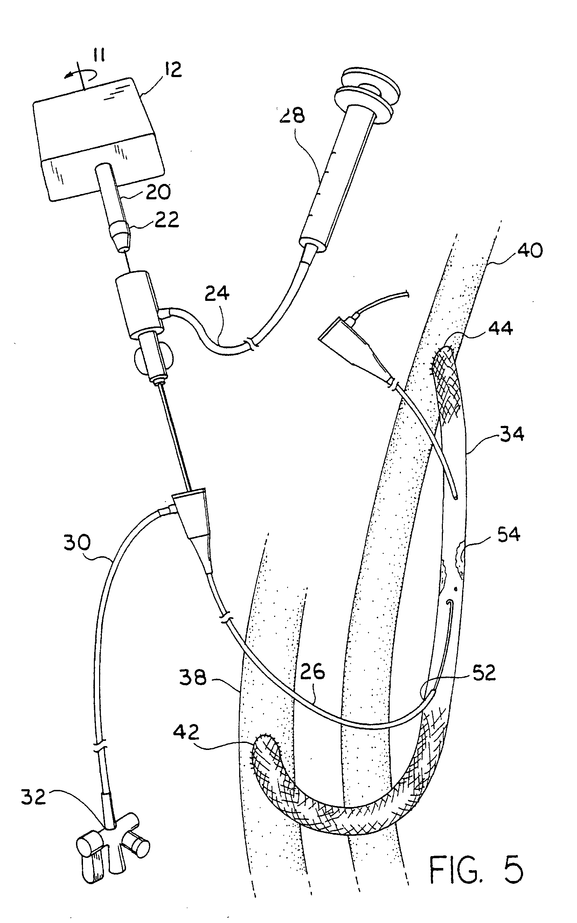

Figure 5 depicts the apparatus in place within a surgical dialysis shunt where the shunt is denoted generally 34 and connects a vein 38 with an artery 40. Vein shunt-juncture is denoted 42 while artery shunt-juncture is denoted 44.

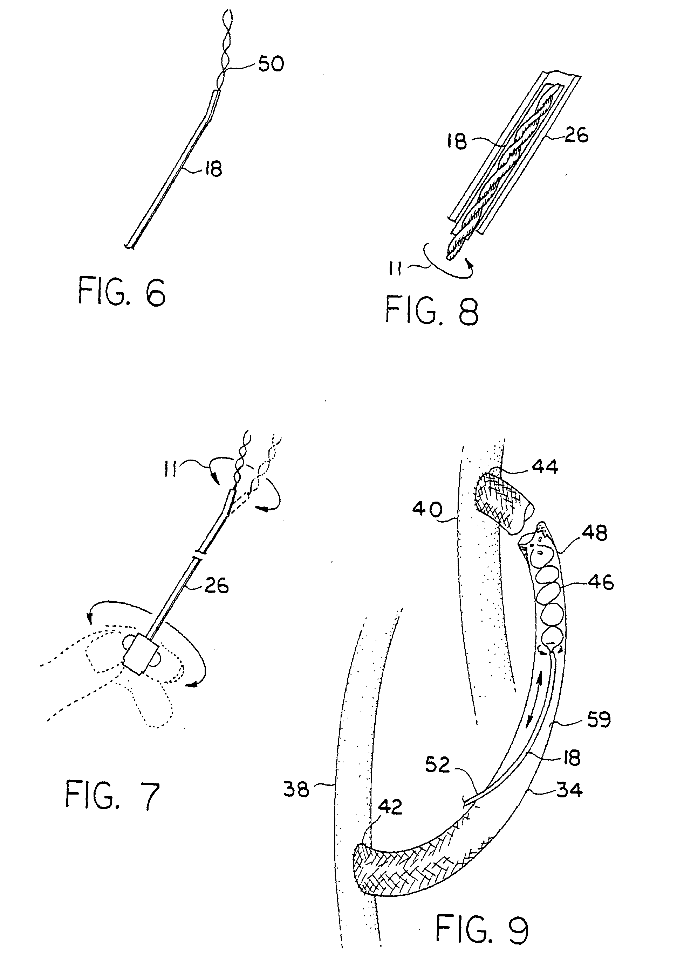

In use of the apparatus for clearing a lumen or shunt of thrombolytic material, rotatable hydrophilic wire 16 is inserted into the interior of the shunt or lumen through a suitable aperture which may be created by puncturing the shunt or lumen with a needle. The wire is then rotated within the lumen at a speed of which the wire forms at least one vibrational node in the portion of the wire within the lumen; this configuration of the wire is depicted in Figure 8. The wire is preferably moved with the vibrational nodes therein axially along the lumen to rotational sweep the thrombolytic material from the lumen; this motion is depicted by arrow A in Figure 8. Preferably not only the wire but also the catheter extremity is inserted into the lumen through the selected aperture. When the wire is rotated, the catheters remain stationary relative to the wire and are manually manipulated in order to guide the wire through the shunt and, as necessary, into the shunt-vein or shunt-artery juncture and, if desired, into the vein or artery to cleanse thrombolytic material therefrom.

The wire is rotated at a speed at which the wire forms at least one vibrational node in the portion 55 of wire 16 extending from catheter 18 into the lumen or shunt. All of this is performed while grasping motor 12 with one hand. Motor 12 preferably has a control by the thumb or forefinger of the hand holding motor 12 so that by using a single hand, the physician or other attending health professional can control not only rotation of wire 16 but also the position of catheter 18 thereby controlling the position of wire 16 within the shunt or other lumen to be cleansed. This frees the second hand of the operator to supply medication or lubricant through conduits 24 or 30 or to perform other activity.

As illustrated in Figure 8, the wire 16 is preferably braided and is rotated in a direction to resist untwisting of the braid.

Manual manipulation of the catheter is illustrated in Figure 7. The angular tip of the catheter 18 when rotated by hand as illustrated permits accurate and close positioning of exposed portion 50 of rotating wire 16. The preferred angular orientation of catheter 18 is illustrated in Figure 6.

Catheter 18 is preferably sufficiently resistive to twisting that the torque manually applied to the catheter proximate to the motor, for example via gripping means 20, causes corresponding angular movement of the extremity of the catheter remote from the motor.

While a braided wire is preferable, a filament wire may be used.

The motor is operated to rotate the wire at a speed to create at least one vibrational node therein. The rotation speed of the wire may be from about 100 revolutions per minute to about 10,000 revolutions per minute. The motor used to turn the wire is desirably electrically powered but may also be pneumatically or hydraulically powered. Also, the wire 16 may be rotated manually if necessary.

Not only does the invention have utility with respect to cleansing of dialysis shunts and the juncture of such shunts with veins and arteries, the invention also has utility in cleansing such arteries and veins of blockages all the way to the heart.

This comprehensive shunt/vein/artery cleansing procedure begins with inserting a needle through skin and into the shunt. The next step is that of inserting a small wire through the needle. The next step is that of using the tactile sensation transmitted by the wire, to determine whether the wire is in the shunt. The next step is that of inspecting the skin site with x-rays to determine the position of the wire and whether it is within the shunt.

The next step is that of removing the needle when the wire is determined to be in the shunt interior. The next step is that of placing a small catheter over the wire with the discharge orifice within the shunt. The next step is that of removing the wire leaving the catheter with its discharge end within the shunt.

The next step is that of inserting a larger wire through the catheter into the shunt interior. The next step is that of removing the catheter. The next step is that of inserting a sheath over the larger wire and into the shunt.

The next step is that of removing the larger second wire. The next step is that of inserting an instrument wire and the catheter through the sheath. The next step is that of supplying lubricating fluid to the catheter interior.

The next step is that of rotating the wire, but not the catheter, and sweeping through the graft to liquify thrombus material therein. The next step is that of removing the instrument wire and the catheter from sheath. The next step is that of applying suction to the sheath to remove liquid thrombus material from the shunt.

The next step is that of inserting an anti-coagulant into the shunt through the sheath. The next step is that of removing the instrument wire from the catheter. The next step is that of disconnecting the instrument wire from the motor.

The next step is that of re-inserting the instrument wire without the catheter through the sheath into the shunt, through any blockage at the shunt end into the vein. The next step is that of placing an angioplasty balloon over the wire. The next step is that of pushing a balloon into position within venous anastomosis at vein-shunt juncture. The next step is that of removing the wire leaving the angioplasty balloon in position.

The next step is that of injecting contrast radiology dye through the balloon lumen vacated by the wire. The next step is that of observing dye travel through the vein to the heart using a fluoroscope revealing any additional venous blockages. The next step is that of inserting a wire back into the balloon lumen. The next step is that of inflating the balloon to crush venous anastomosis and open the shunt-vein juncture.

The next step is that of removing the balloon and wire. The next step is that of inserting a second sheath between position of the first sheath insertion and shunt-vein juncture, into a clean shunt region. The next step is that of re-inserting the instrument wire without the catheter through the sheath into the shunt, through any blockage at the shunt-artery juncture. The next step is that of placing an angioplasty balloon over the wire.

The next step is that of pushing the balloon into position within arterial anastomosis at artery-shunt juncture. The next step is that of removing the wire leaving the angioplasty balloon in position. The next step is that of injecting contrast radiology dye through the balloon lumen vacated by the wire.

The next step is that of observing dye travel through the artery to the heart using a fluoroscope and revealing any additional arterial blockages. The next step is that of inserting a wire back into the balloon lumen.

The next step is that of inflating the balloon to crush any arterial anastomosis and open the shunt-artery juncture. The next step is that of removing a platelet plug and residual arterial anastomosis from the shunt-artery juncture by pulling on the balloon. The final step is that of removing the balloon, wire and the sheath.

## Claims

1. Apparatus for clearing a body lumen of thrombolytic material comprising one hand manipulable means (12) for providing rotary motion output, an elongated wire (16) connected to the rotary motion output means (12) for rotation thereby, a catheter (18) for enveloping a length of the wire, and gripping means (20) facilitating manual rotation of the catheter by the one hand independently of the wire, **characterised in that** the elongated wire (16) is flexible and the rotary motion output means (12) is adapted to rotate the wire at a speed sufficient to cause the wire to flex and to create a standing wave in a portion of the wire extending from the catheter to liquefy thrombolytic material.

2. Apparatus as claimed in claim 1, **characterised in that** the means (12) for providing rotary motion output comprises a motor (12) including control means.

3. Apparatus as claimed in claim 2, **characterised in that** a housing is provided for the motor (12) and for the control means, adapted for grasping by the one hand, the wire (16) passing through the housing and emerging from opposite sides thereof.

4. Apparatus as claimed in any preceding claim, **characterised in that** the extremity of the wire (16) remote from the catheter (18) is angularly disposed with respect to the remainder of the wire.

5. Apparatus as claimed in any one of claims 1 to 3, **characterised in that** the extremity of the wire (16) remote from the catheter (18) is J-shaped.

6. Apparatus as claimed in any one of claims 1 to 3, **characterised in that** the extremity of the catheter (18) remote from the means (12) for providing rotary motion output is angularly disposed with respect to the remainder of the catheter.

7. Apparatus as claimed in any preceding claim, **characterised in that** the catheter (18) is sufficiently resistant to twisting that torque applied manually to the catheter proximate the means (12) for providing rotary motion output results in corresponding angular rotary motion of the extremity of the catheter remote from the means (12).

8. Apparatus as claimed in any preceding claim, **characterised in that** means (24) is provided communicating with the interior of the catheter (18) for selectably supplying or exhausting fluid to and from the catheter interior.

9. Apparatus as claimed in claim 8, **characterised in that** the gripping means (20) is fixedly connected to the means (24) communicating with the interior of the catheter (18).

10. Apparatus as claimed in claim 9, **characterised in that** the gripping means (20) is rotatable unitarily with the catheter (18) about the wire (16).

11. Apparatus as claimed in any preceding claim, **characterised in that** a sheath (26) is provided enveloping the catheter (18) and a conduit (30) communicating with the interior of the sheath for selectably supplying or exhausting fluid to and from the sheath interior, externally of the catheter.

12. Apparatus as claimed in claim 11, **characterised in that** the conduit (30) is movable longitudinally along the wire with the sheath (26).

13. Apparatus as claimed in claim 11 or 12, when dependent on claim 8, 9 or 10, **characterised in that** the means (12) providing rotary motion output, the catheter (18), the wire (16), the means (24) communicating with the interior of the catheter, the sheath (26) and the conduit (30) communicating with the interior of the sheath are all manually disassemblable from one another.

14. Apparatus as claimed in claim 13, **characterised in that** the components (12, 18, 16, 24, 26, 30) are manually disassemblable without the use of tools.

15. Apparatus as claimed in any preceding claim, **characterised in that** manually actuable means (22) is provided for selectably connecting the wire (16) to the means (12) for providing rotary motion output.

16. Apparatus as claimed in any preceding claim, **characterised in that** the wire (16) is hydrophilic.

17. Apparatus as claimed in any preceding claim, **characterised in that** the wire (16) is braided.

18. Apparatus as claimed in claim 17, **characterised in that** the wire (16) is rotatable in a direction to resist untwisting of the braid.

19. Apparatus as claimed in any one of claims 1 to 16, **characterised in that** the wire (16) is a single filament.

20. Apparatus as claimed in any preceding claim, **characterised in that** the wire (16) is not permanently deformable at room temperature.

21. Apparatus as claimed in any preceding claim, **characterised in that** the wire (16) is rotatable at a speed from 100 revolutions per minute to 10,000 revolutions per minute.

## Patentansprüche

1. Vorrichtung zum Beseitigen von thrombolytischem Material aus einem Körperlumen, umfassend ein mit einer Hand zu bedienendes Mittel (12) zum Erzeugen eines Drehbewegungsausgangs, einen länglichen Draht (16), der mit dem Mittel (12) zum Erzeugen eines Drehbewegungsausgangs verbunden ist, um von diesem gedreht zu werden, einen Katheter (18) zum Umhüllen eines Stücks des Drahtes, und ein Greifmittel (20), das die manuelle Drehung des Katheters mit der einen Hand unabhängig von dem Draht erleichtert, **dadurch gekennzeichnet, dass** der längliche Draht (16) flexibel ist und das Mittel (12) zum Erzeugen eines Drehbewegungsausgangs die Aufgabe hat, den Draht mit einer Geschwindigkeit zu drehen, die ausreicht, um zu bewirken, dass sich der Draht biegt und dass eine Stehwelle in einem Abschnitt des Drahtes entsteht, der sich von dem Katheter erstreckt, um thrombolytisches Material zu verflüssigen.

2. Vorrichtung nach Anspruch 1, **dadurch gekennzeichnet, dass** das Mittel (12) zum Erzeugen eines Drehbewegungsausgangs einen Motor (12) mit einem Steuermittel umfasst.

3. Vorrichtung nach Anspruch 2, **dadurch gekennzeichnet, dass** ein Gehäuse für den Motor (12) und für das Steuermittel vorgesehen ist, das so gestaltet ist, dass es mit der einen Hand erfasst wird, wobei der Draht (16) durch das Gehäuse passiert und auf den gegenüberliegenden Seiten davon austritt.

4. Vorrichtung nach einem der vorherigen Ansprüche, **dadurch gekennzeichnet, dass** das Ende des Drahtes (16) fern von dem Katheter (18) einen Winkel mit dem Rest des Drahtes bildet.

5. Vorrichtung nach einem der Ansprüche 1 bis 3, **dadurch gekennzeichnet, dass** das Ende des Drahtes (16) fern von dem Katheter (18) J-förmig ist.

6. Vorrichtung nach einem der Ansprüche 1 bis 3, **dadurch gekennzeichnet, dass** das Ende des Katheters (18) fern von dem Mittel (12) zum Erzeugen eines Drehbewegungsausgangs einen Winkel mit dem Rest des Katheters bildet.

7. Vorrichtung nach einem der vorherigen Ansprüche, **dadurch gekennzeichnet, dass** der Katheter (18) so verdrehfest ist, dass ein manuell auf den Katheter in der Nähe des Mittels (12) zum Erzeugen eines Drehbewegungsausgangs aufgebrachtes Drehmoment in einer entsprechenden winkelmäßigen Drehbewegung des Endes des Katheters fern von dem Mittel (12) resultiert.

8. Vorrichtung nach einem der vorherigen Ansprüche, **dadurch gekennzeichnet, dass** Mittel (24) zum Kommunizieren mit dem Inneren des Katheters (18) zum selektiven Zuführen oder Ablassen von Fluid zu und von dem Katheterinneren vorgesehen sind.

9. Vorrichtung nach Anspruch 8, **dadurch gekennzeichnet, dass** das Greifmittel (20) fest mit dem Mittel (24) verbunden ist, das mit dem Inneren des Katheters (18) in Verbindung steht.

10. Vorrichtung nach Anspruch 9, **dadurch gekennzeichnet, dass** das Greifmittel (20) einheitlich mit dem Katheter (18) um den Draht (16) gedreht werden kann.

11. Vorrichtung nach einem der vorherigen Ansprüche, **dadurch gekennzeichnet, dass** eine Hülle (26) vorgesehen ist, die den Katheter (18) umgibt, und eine Leitung (30), die mit dem Inneren der Hülle in Verbindung ist, um selektiv Fluid zu oder von dem Hülleninneren außerhalb des Katheters zuzuführen oder abzulassen.

12. Vorrichtung nach Anspruch 11, **dadurch gekennzeichnet, dass** die Leitung (30) longitudinal entlang des Drahtes mit der Hülle (26) beweglich ist.

13. Vorrichtung nach Anspruch 11 oder 12 in Abhängigkeit von Anspruch 8, 9 oder 10, **dadurch gekennzeichnet, dass** das Mittel (12) zum Erzeugen eines Drehbewegungsausgangs, der Katheter (18), der Draht (16), das Mittel (24) in Verbindung mit dem Inneren des Katheters, die Hülle (26) und die Leitung (30) in Verbindung mit dem Innern der Hülle alle manuell zerlegt werden können.

14. Vorrichtung nach Anspruch 13, **dadurch gekennzeichnet, dass** die Komponenten (12, 18, 16, 24, 26, 30) manuell ohne Zuhilfenahme von Werkzeugen zerlegt werden können.

15. Vorrichtung nach einem der vorherigen Ansprüche, **dadurch gekennzeichnet, dass** das manuell zu betätigende Mittel (22) zum selektiven Verbinden des Drahtes (16) mit dem Mittel (12) zum Erzeugen eines Drehbewegungsausgangs vorgesehen ist.

16. Vorrichtung nach einem der vorherigen Ansprüche, **dadurch gekennzeichnet, dass** der Draht (16) hydrophil ist.

17. Vorrichtung nach einem der vorherigen Ansprüche, **dadurch gekennzeichnet, dass** der Draht (16) geflochten ist.

18. Vorrichtung nach Anspruch 17, **dadurch gekennzeichnet, dass** der Draht (16) in einer Richtung drehbar ist, die ein Entflechten verhindert.

19. Vorrichtung nach einem der Ansprüche 1 bis 16, **dadurch gekennzeichnet, dass** der Draht (16) ein einzelnes Filament ist.

20. Vorrichtung nach einem der vorherigen Ansprüche, **dadurch gekennzeichnet, dass** der Draht (16) bei Raumtemperatur nicht permanent verformbar ist.

21. Vorrichtung nach einem der vorherigen Ansprüche, **dadurch gekennzeichnet, dass** der Draht (16) mit einer Drehzahl von 100 Umdrehungen pro Minute bis zu 10.000 Umdrehungen pro Minute gedreht werden kann.

## Revendications

1. Dispositif pour enlever des matériaux thrombolytiques dans une lumière du corps, comprenant un moyen manipulable à la main pour fournir une source de mouvement rotatif (12), un fil métallique allongé (16) connecté à la source de mouvement rotatif (12) pour le faire tourner, un cathéter (18) enveloppant une section du fil métallique, et un moyen de préhension (20) facilitant la rotation manuelle d'une seule main du cathéter indépendamment du fil métallique, **caractérisé en ce que** le fil métallique allongé (16) est flexible et la source de mouvement rotatif (12) est adaptée de façon à faire tourner le fil à une vitesse suffisante pour le cintrer et créer une onde stationnaire dans une partie du fil sortant du cathéter afin de liquéfier le matériau thrombolytique.

2. Dispositif selon la revendication 1, **caractérisé en ce que** le moyen pour fournir la source de mouvement rotatif (12) comprend un moteur (12) avec dispositif de commande.

3. Dispositif selon la revendication 2, **caractérisé en ce qu'**un boîtier est fourni pour le moteur (12) et son moyen de commande, adapté afin de pouvoir le saisir d'une main, le fil métallique (16) passant à travers le boîtier et émergeant de côtés opposés de celui-ci.

4. Dispositif selon n'importe laquelle des revendications précédentes, **caractérisé en ce que** l'extrémité du fil (16) distale par rapport au cathéter (18) forme un angle par rapport au reste du fil.

5. Dispositif selon n'importe laquelle des revendications 1 à 3, **caractérisé en ce que** l'extrémité du fil (16) distale par rapport au cathéter (18) est en forme de J.

6. Dispositif selon n'importe laquelle des revendications 1 à 3, **caractérisé en ce que** l'extrémité du cathéter (18) distale par rapport au moyen pour fournir la source de mouvement rotatif (12) est disposée selon un angle par rapport au reste du cathéter.

7. Dispositif selon n'importe laquelle des revendications précédentes , **caractérisé en ce que** le cathéter (18) est suffisamment résistant aux torsions pour que le couple appliqué manuellement au cathéter du côté proximal du moyen pour fournir la source de mouvement rotatif (12) entraîne un mouvement rotatif angulaire correspondant de l'extrémité du cathéter distale de cette source (12).

8. Dispositif selon n'importe laquelle des revendications précédentes, **caractérisé en ce qu'**un moyen (24) en communication avec l'intérieur du cathéter (18) est fourni pour apporter dans ou retirer sélectivement un fluide de l'intérieur du cathéter.

9. Dispositif selon la revendication 8, **caractérisé en ce que** le moyen de préhension (20) est connecté de manière fixe au moyen (24) communiquant avec l'intérieur du cathéter (18).

10. Dispositif selon la revendication 9, **caractérisé en ce que** le moyen de préhension (20) peut être tourné individuellement avec le cathéter (18) autour du fil (16).

11. Dispositif selon n'importe laquelle des revendications précédentes, **caractérisé en ce qu'**une gaine (26) enveloppant le cathéter (18) est fournie ainsi qu'un conduit (30) communiquant avec l'intérieur de celle-ci pour apporter dans ou retirer sélectivement un fluide de l'intérieur de la gaine, à l'extérieur du cathéter.

12. Dispositif selon la revendication 11, **caractérisé en ce que** le conduit (30) peut être déplacé longitudinalement le long du fil avec la gaine (26).

13. Dispositif selon la revendication 11 ou 12, lorsque cela dépend de la revendication 8, 9 ou 10, **caractérisé en ce que** la source de mouvement rotatif (12), le cathéter (18), le fil métallique (16), le moyen (24) de communiquer avec l'intérieur du cathéter, la gaine (26) et le conduit (30) communiquant avec l'intérieur de celle-ci sont tous démontables manuellement l'un par rapport à l'autre.

14. Dispositif selon la revendication 13, **caractérisé en ce que** les composants (12, 18, 16, 24, 26, 30) sont démontables manuellement sans avoir à utiliser d'outils.

15. Dispositif selon n'importe laquelle des revendications précédentes, **caractérisé en ce que** le moyen actionnable manuellement (22) est fourni pour connecter de manière sélective le fil (16) à la source de mouvement rotatif (12).

16. Dispositif selon n'importe laquelle des revendications précédentes, **caractérisé en ce que** le fil métallique (16) est hydrophile.

17. Dispositif selon n'importe laquelle des revendications précédentes, **caractérisé en ce que** le fil métallique (16) est tressé.

18. Dispositif selon la revendication 17, **caractérisé en ce que** le fil (16) est mis en rotation dans un sens de façon à éviter de détorsader le tressage.

19. Dispositif selon n'importe laquelle des revendications 1 à 16, **caractérisé en ce que** le fil métallique (16) est un monofilament.

20. Dispositif selon n'importe laquelle des revendications précédentes, **caractérisé en ce que** le fil métallique (16) n'est pas déformable de manière permanente à la température ambiante.

21. Dispositif selon n'importe laquelle des revendications précédentes, **caractérisé en ce que** le fil métallique (16) peut être mis en rotation à une vitesse allant de 100 tours par minute à 10 000 tours par minute.
